# EUROPÄISCHE PATENTSCHRIFT

(11) **EP 0 600 460 B1**
(45) Veröffentlichungstag und Bekanntmachung des Hinweises auf die Patenterteilung: **17.02.1999**
(21) Anmeldenummer: 93119354.4
(22) Anmeldetag: 01.12.1993
(51) Int. Cl.: C07D 475/04, C07D 487/14

(54) **Verfahren zur Herstellung von (6S)-5,6,7,8-Tetrahydrofolsäure**
Process for the preparation of 6(S)-5,6,7,8-tetrahydrofolic acid
Procédé pour la préparation du l'acide 6(S)-5,6,7,8-tétrahydrofolique

(30) Priorität: 01.12.1992 CH 3674/92
(43) Veröffentlichungstag der Anmeldung: 08.06.1994
(73) Patentinhaber: Cerbios-Pharma S.A., CH-6917 Barbengo (CH)
(72) Erfinder: Jéquier, Pascal, CH-6815 Melide (CH); Marazza, Fabrizio, CH-6986 Novaggio (CH)
(74) Vertreter: Zink-Wild, Markus Peter

(56) Entgegenhaltungen:
- EP-A- 0 293 029
- EP-A- 0 348 641
- EP-A- 0 367 902
- EP-A- 0 455 013
- EP-A- 0 495 204
- WO-A-91/13890

## Beschreibung

Die vorliegende Erfindung betrifft ein Verfahren zur Herstellung von (6S)-5,6,7,8-Tetrahydrofolsäure.

5,6,7,8-Tetrahydrofolsäure wird hierin manchmal mit THF abgekürzt.

Eine Synthese von diastereoisomerenreinen reduzierten Folaten sowie deren Bedeutung im pharmazeutischen Bereich sind in der europäischen Patentanmeldung Nr. 91 905 831.3-2101, Veröffentlichungs-Nr. 0 471 820 beschrieben.

Mit diesen Hinweis werden die in dieser Veröffentlichung enthaltenen Aussagen als hierin offenbart betrachtet.

In der europäischen Patentanmeldung Nr. 91 121 326.2, Veröffentlichungs-Nr. 0 495 204 A1 wird unter anderem ein Verfahren zur Herstellung von (6S)- und (6R)-Tetrahydrofolsäure via die fraktionierte Kristallisation von entsprechenden Säureadditionssalzen mit einer Sulfonsäure oder mit Schwefelsäure beschrieben.

In dieser Publikation wird nichts von einer direkten, selektiven Kristallisation der (6S)-THF in der Form der freien Säure erwähnt.

Das in EP 0 495 204 A1 beschriebene Verfahren beinhaltet unnötige Stufen, nämlich die Herstellung von Säureadditionssalzen und deren Umwandlung in die freie Säure.

Die fraktionierte Kristallisation dieser Säureadditionssalze muss offenbar gemäss den in den Beispielen beschriebenen Ausführungsformen stets in Gegenwart eines Antioxidationsmittels, wie etwa Mercaptoethanol, durchgeführt werden.

Dieses Verfahren ist nicht wirtschaftlich und nicht umweltfreundlich, da als Nebenprodukte bei der Freisetzung der Säure aus dem Säureadditionssalz mindestens ein Aequivalent eines Sulfonsäure- oder Schwefelsäuresalzes entsteht, welches beseitigt werden muss.

Eine direkte, selektive Kristallisation von (6S)-THF in der Form der freien Säure ist bis jetzt noch nicht beschrieben.

Es ist ein Ziel der vorliegenden Erfindung, ein Verfahren zur Herstellung von (6S)-THF zur Verfügung zu stellen, bei dem eine direkte, selektive Kristallisation von (6S)-THF in der Form der freien Säure verwendet wird.

Völlig überraschend wurde gefunden, dass innerhalb von bestimmten pH- und Temperaturbereichen (6S)-THF selektiv kristallisiert werden kann.

Das erfindungsgemässe Verfahren zur Herstellung von (6S)-5,6,7,8-Tetrahydrofolsäure- abgekürzt mit (6S)-THF - der Formel I 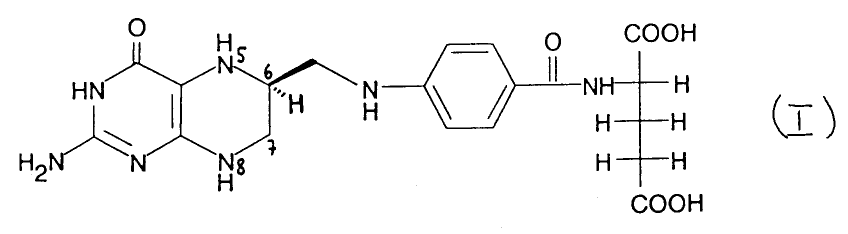 mit einer diastereoisomeren Reinheit von wenigstens 75 % ist dadurch gekennzeichnet, dass man zu einer wässrigen Lösung eines (6RS)-Diastereoisomerengemisches eines Alkalimetallsalzes von 5,6,7,8-Tetrahydrofolsäure bei einer Temperatur von 5°C bis 80°C zwecks selektiver Kristallisation so lange portionenweise Säure hinzugibt, bis sich der pH-Wert auf einen Wert im Bereich von 4,8 bis 5,3 stabilisiert hat, und den entstandenen Festkörper, nämlich (6S)-5,6,7,8-Tetrahydrofolsäure, abtrennt.

Bevorzugte Ausführungsformen dieser Erfindung sind in den abhängigen Ansprüchen definiert.

Das im erfindungsgemässen Verfahren verwendete Ausgangsmaterial, beispielsweise das Dinatriumsalz von (6RS)-THF, kann gemäss E. Khalifa et. al., Helv. (1980), 63, 2554 hergestellt werden.

Dieses Ausgangsmaterial kann man auch dadurch erhalten, indem man das (6RS)-Diastereoisomerengemisch von THF, vorzugsweise in Gegenwart des Dinatriumsalzes von Ethylendiamintetraessigsäure (EDTA), unter einer Inertgasatmosphäre bei einer Temperatur von etwa 35°C im Wasser suspendiert, und solange wässrige Natronlauge zutropft, bis man eine homogene Lösung erhält. Diese Lösung hat normalerweise einen pH-Wert von etwa 6,5.

Zwecks Stabilitätserhöhung ist es vorteilhaft, den pH-Wert dieser Lösung auf einen Wert von 5,5 bis 5,0 mittels der Hinzugabe einer der genannten bevorzugten Säuren einzustellen.

Danach wird diese Lösung vorzugsweise auf eine Temperatur von 40°C bis 50°C, insbesondere 45°C, erwärmt.

Anschliessend wird zwecks selektiver Kristallisation von (6S)-THF so lange Säure hinzugetropft, bis sich der pH-Wert auf einen Wert im Bereich von 4,8 bis 5,3, vorzugsweise 4,9 bis 5,2, insbesondere 5,0, stabilisiert hat.

Man beobachtet die Bildung eines kristallinen Festkörpers während dieser Säurehinzugabe.

Der kristalline Festkörper wird abgetrennt, beispielsweise mittels Filtration oder Zentrifugation, und in üblicher Art gewaschen und getrocknet.

Generell wurde festgestellt, dass bei einem relativ tiefen pH-Wert, beispielsweise 4,5, eine geringere Kristallisationsselektivität (6S) versus (6R) verbunden mit einer relativ hohen Ausbeute erhalten wird.

Bei einem höheren pH-Wert, beispielsweise 5,0, ist die Kristallisationsselektivität besser, aber die Ausbeute ist geringer.

Entsprechende genaue Daten sind aus der folgenden Tabelle ersichtlich:

| pH-Wert | Temperatur | Verhältnis (6S)/(6R)-THF | Ausbeute % |
|---|---|---|---|
| 5,2 | 35°C | 80 : 20 | 45 |
| 5,0 | 25°C (14h) | 77 : 23 | 54 |
| 4,8 | 40°C | 75 : 25 | 54 |
| 4,5 | 45°C | 67 : 33 | 67 |
| 5,0 | 50°C | 80 : 20 | 48 |
| 4,5 | 70°C | 64 : 36 | 55 |
| 5,0 | 45°C | 80,5: 19,5 | 48 |

Die selektive Kristallisation ist normalerweise nach höchstens 1 Stunde abgeschlossen.

Für die Gewinnung von diastereoisomerenreiner (6S)-THF kann das erfindungsgemässe Verfahren mehrmals hintereinander durchgeführt werden.

Die folgende Tabelle beschreibt die Umkristallisation eines Diastereoisomerengemisches von 80% (6S)- und 20 % (6R)-THF bei verschiedenen Temperaturen und pH-Werten:

| pH-Wert | Temperatur | Verhältnis (6S)/(6R)-THF | Ausbeute % |
|---|---|---|---|
| 5,0 | 50°C | 89: 11 | 86 |
| 5,6 | 25°C | 94: 6 | 66 |
| 5,2 | 45°C | 92: 8 | 75 |

(6R)-THF kann, falls gewünscht, aus der Mutterlauge erhalten werden.

Dazu wird ein Aequivalent eines Calciumsalzes, beispielsweise ein Calciumhalogenid, wie Calciumchlorid und Calciumbromid, Calciumacetat, bezogen auf die anwesende Menge an THF, als Festkörper zur Mutterlauge gegeben.

Anschliessend gibt man eine Base, beispielsweise wässrige Natronlauge, hinzu, bis man einen pH-Wert von etwa 6,0 erhält.

Die Lösung wird teilweise eingeengt, beispielsweise auf eine Konzentration von 10 % an THF in der eingeengten Lösung.

Aus dieser eingeengten Lösung kristallisiert in etwa 2 Stunden (6R)-THF in der Form des Calciumsalzes aus.

Die nachfolgenden Beispiele illustrieren die vorliegende Erfindung.

### Beispiel 1

100,0 g (6RS)-5,6,7,8-Tetrahydrofolsäure, hergestellt nach E. Khalifa et al. Helv., (1980), 63, 2554, und 2,0 g des Dinatrium Salzes der Ethylendiamintetraessigsäure (EDTA) wurden unter einer Stickstoffatmosphäre bei einer Temperatur von 35°C in 750 ml Wasser suspendiert.

Es wurde solange 20%-ige wässrige Natronlauge hinzugetropft, bis man eine homogene Lösung erhielt, welche einen pH-Wert von 6,5 zeigte.

Dann wurde das Gemisch stark gerührt. Durch tropfenweise Zugabe von 18%-iger, wässriger Salzsäure wurde der pH-Wert bis auf einen Wert von 4,8 gebracht. Die Temperatur wurde anschliessend auf 45°C erhöht.

Nach einigen Minuten bei der genannten Temperatur beobachtete man die Bildung eines kristallinen Festkörpers, wobei gleichzeitig der pH-Wert der Suspension zu steigen begann.

Durch Zugabe von 18%-iger wässriger Salzsäure wurde der pH-Wert bei 5,0 gehalten, bis sich nach ungefähr 45 Minuten dieser Wert stabilisiert hatte.

Der weisse, kristalline Festkörper wurde mittels Filtration isoliert, einmal mit Wasser und einmal mit Aceton gewaschen und dann unter vermindertem Druck bei einer Temperatur von 50°C getrocknet.

Auf diese Weise erhielt man 48 g an kristalliner 5,6,7,8-Tetrahydrofolsäure.

Zur Bestimmung des Diastereoisomeren-Verhältnisses wurde eine Probe des erhaltenen Produktes gemäss K.O. Donaldson und Keresztesy J.C., J. Biol. Chem., (1962), 237, 3815 oder V.S. Gupta und F.M. Huennekens, Arch. Biochem. Biophys. (1967), 120, 712 in N(5)-CH₃-THF umgewandelt.

Das so erhaltene Produkt wurde an einer chiralen Säule (RESOLVOSIL) analysiert.

Es wurde ein Diastereoisomeren-Verhältnis von (6S)/(6R) = 80.5/19.5 ermittelt.
HPLC-Analyse (reverse phase-Säule): 99,3 %
Wassergehalt nach Karl Fischer : 7.5 %
Chlorid Gehalt : 0 %

### Beispiel 2

40 g des gemäss Beispiel 1 erhaltenen Produktes wurden in 500 ml Wasser suspendiert und in Analogie zu Beispiel 1 behandelt.

Die selektive Kristallisation der freien Säure erfolgte bei einem pH-Wert von 5,2.

Man erhielt 30,2 g an kristalliner 5,6,7,8-Tetrahydrofolsäure mit einem Diastereoisomeren-Verhältnis von (6S)/(6R) = 93 / 7.
HPLC-Analyse (reverse phase-Säule) : 99,1 %
Wassergehalt nach Karl Fischer: 5,7 %
[α]_{D} = -32,5° (c=1, 0,04 N NaOH, pH = 9,5)
Chlorid Gehalt : 0 %

| Elementaranalyse | berechnet (%) | gefunden (%) |
|---|---|---|
| C₁₉H₂₃N₇O₆.1,5 H₂O | C 48,32 | 48,59 |
| | N 20,76 | 21,06 |
| | H 5,55 | 5,57. |

## Patentansprüche

1. Verfahren zur Herstellung von (6S)-5,6,7,8-Tetrahydrofolsäure- abgekürzt mit (6S)-THF - der Formel I 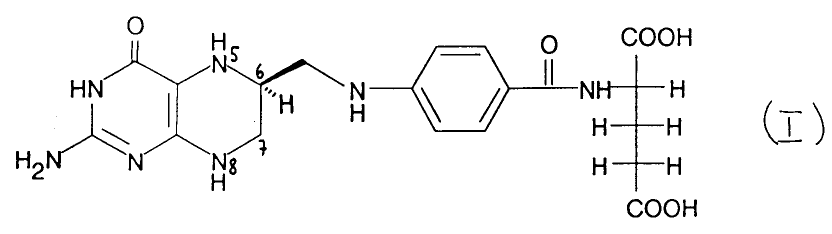 mit einer diastereoisomeren Reinheit von wenigstens 75 %,
dadurch gekennzeichnet, dass man zu einer wässrigen Lösung eines (6RS)-Diastereoisomerengemisches eines Alkalimetallsalzes von 5,6,7,8-Tetrahydrofolsäure bei einer Temperatur von 5°C bis 80°C zwecks selektiver Kristallisation so lange portionenweise Säure hinzugibt, bis sich der pH-Wert auf einen Wert im Bereich von 4,8 bis 5,3 stabilisiert hat, und den entstandenen Festkörper, nämlich (6S)-5,6,7,8-Tetrahydrofolsäure, abtrennt.

2. Verfahren nach Anspruch 1, dadurch gekennzeichnet, dass die genannte wässrige Lösung des (6RS)-Diastereoisomerengemisches noch wenigstens ein Komplexierungsmittel für Schwermetalle enthält, vorzugsweise ausgewählt aus der Gruppe, bestehend aus Ethylen-diamintetraessigsäure, abgekürzt mit EDTA, und deren Salze und Hydrate, insbesondere das di-Natriumsalz, Nitrilotriessigsäure, 1,2-Diaminocyclohexan-N,N,N',N'-tetraessigsäure, wobei EDTA und deren di-Natriumsalz bevorzugt sind.

3. Verfahren nach einem der Ansprüche 1 bis 2, dadurch gekennzeichnet, dass die Zugabe von Säure und die gleichzeitig stattfindende selektive Kristallisation bei einer Temperatur von 40°C bis 50°C erfolgt.

4. Verfahren nach einem der Ansprüche 1 bis 3, dadurch gekennzeichnet, dass die Säure ausgewählt ist aus der Gruppe, bestehend aus Halogenwasserstoffsäuren, insbesondere Salzsäure, Schwefelsäure, Phosphorsäure, Essigsäure, Trichloressigsäure, Trifluoressigsäure, Methansulfonsäure, p-Toluolsulfonsäure, insbesondere wässrige Lösungen dieser Säuren.

5. Verfahren nach einem der Ansprüche 1 bis 4, dadurch gekennzeichnet, dass man den pH-Wert auf einen Wert im Bereich von 4,9 bis 5,2, insbesondere 5,0, stabilisiert.

6. Verfahren nach einem der Ansprüche 1 bis 5, dadurch gekennzeichnet, dass die ganze Reaktion unter Inertgasatmosphäre ausgeführt wird.

## Claims

1. A process for the preparation of (6S)-5,6,7,8-tetrahydrofolic acid - abbreviated with (6S)-THF - of the formula I 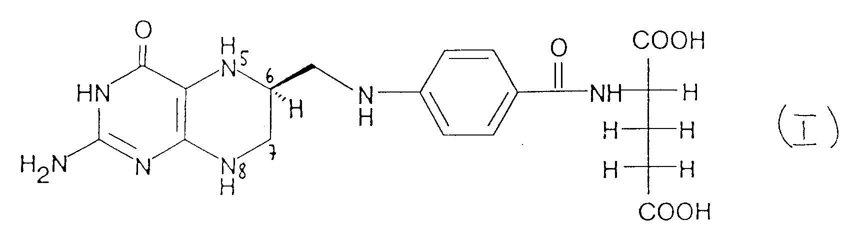 having a diastereoisomeric purity of at least 75 %,
characterized by adding in portions acid to an aqueous solution of a (6RS)-diastereoisomeric mixture of an alkali metal salt of 5,6,7,8-tetrahydrofolic acid at a temperature from 5°C to 80°C for the purpose of the selective crystallization during such a long time until the pH-value has stabilized to a value in the range from 4.8 to 5.3, and separating the formed solid, that is (6S)-5,6,7,8-tetrahydrofolic acid.

2. The process according to claim 1, characterized in that said aqueous solution of the (6RS)-diastereoisomeric mixture contains additionally at least one complexing agent for heavy metals, preferably selected from the group, consisting of ethylene-diamine-tetraacetic acid, abbreviated with EDTA, and its salts and hydrates, especially the di-sodium salt, nitrilotriacetic acid, 1,2-diaminocyclohexane-N,N,N',N'-tetraacetic acid, whereby EDTA and its di-sodium salt are preferred.

3. The process according to one of claims 1 to 2, characterized in that the addition of acid and the simultaneously occuring selective crystallization are realized at a temperature from 40°C to 50°C.

4. The process according to one of claims 1 to 3, characterized in that the acid is selected from the group, consisting of hydrohalic acids, especially hydrochloric acid, sulfuric acid, phosphoric acid, acetic acid, trichloroacetic acid, trifluoroacetic acid, methanesulfonic acid, p-toluenesulfonic acid, especially aqueous solutions of these acids.

5. The process according to one of claims 1 to 4, characterized in that the pH-value is stabilized to a value in the range from 4.9 to 5.2, especially 5.0.

6. The process according to one of claims 1 to 5, characterized in that the whole reaction is carried out under inert gas atmosphere.

## Revendications

1. Procédé pour la préparation de l'acide (6S)-5,6,7,8-tétrahydrofolique - en abrégé (6S)-THF - répondant à la formule I 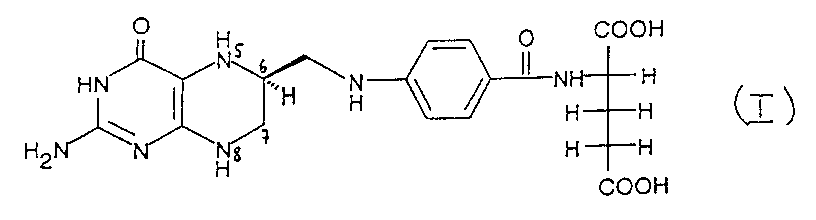 possédant une pureté diastéréo-isomérique d'au moins 75%,
caractérisé en ce qu'on ajoute, par portions, de l'acide à une solution aqueuse d'un mélange diastéréoisomérique (6RS) d'un sel de métal alcalin de l'acide 5,6,7,8-tétrahydrofolique, à une température de 5°C à 80°C, à des fins de cristallisation sélective, jusqu'à la stabilisation du pH à une valeur dans le domaine de 4,8 à 5,3, et on sépare le corps solide obtenu, à savoir l'acide (6S)-5,6,7,8-tétrahydrofolique.

2. Procédé selon la revendication 1, caractérisé en ce que la solution aqueuse mentionnée du mélange de diastéréo-isomères (6RS) contient encore au moins un agent de complexation pour des métaux lourds, de préférence choisi parmi le groupe constitué par l'acide éthylènediaminetétracétique, en abrégé EDTA, ainsi que ses sels et ses hydrates, en particulier le sel disodique, l'acide nitrilotriacétique, l'acide 1,2-diaminocyclohexane-N,N,N',N'-tétraacétique, le EDTA et son sel disodique étant préférés.

3. Procédé selon l'une quelconque des revendications 1 à 2, caractérisé en ce qu'on procède à l'addition de l'acide et à la cristallisation sélective qui se déroulent de manière simultanée, à une température de 40°C à 50°C.

4. Procédé selon l'une quelconque des revendications 1 à 3, caractérisé en ce qu'on sélectionne l'acide parmi le groupe constitué par des acides halogénhydriques, en particulier l'acide chlorhydrique, l'acide sulfurique, l'acide phosphorique, l'acide acétique, l'acide trichloracétique, l'acide trifluoracétique, l'acide méthanesulfonique, l'acide p-toluènesulfonique, en particulier des solutions aqueuses de ces acides.

5. Procédé selon l'une quelconque des revendications 1 à 4, caractérisé en ce qu'on stabilise le pH à une valeur dans le domaine de 4,9 à 5,2, en particulier à une valeur de 5,0.

6. Procédé selon l'une quelconque des revendications 1 à 5, caractérisé en ce qu'on effectue toute la réaction sous l'atmosphère d'un gaz inerte.
